# EUROPEAN PATENT SPECIFICATION

(11) **EP 2 501 424 B1**
(45) Date of publication and mention of the grant of the patent: **21.03.2018**
(21) Application number: 10831862.7
(22) Date of filing: 16.11.2010
(51) Int. Cl.: A61M 16/00, A61M 16/04

(54) **TRACHEOSTOMY VALVE**
TRACHEOSTOMIEKLAPPE
SOUPAPE DE TRACHÉOTOMIE

(30) Priority: 17.11.2009 SE 0901460
(43) Date of publication of application: 26.09.2012
(73) Proprietor: Fogless International AB, 551 12 Jönköping (SE)
(72) Inventor: EKEBERG, Daniel, S-551 12 Jönköping (SE); BLOMQUIST, Inge, S-567 32 Vaggeryd (SE)
(74) Representative: Zacco Sweden AB
(86) International application number: PCT/SE2010/000274
(87) International publication number: WO 2011/062533

(56) References cited:
- EP-A1- 0 078 685
- WO-A1-00/30708
- WO-A1-02/074377
- WO-A1-2009/018384
- WO-A1-2009/018384
- WO-A2-2008/132222
- US-A- 5 806 515
- US-A1- 2004 123 868
- US-A1- 2006 260 703

## Description

### Field of the invention.

The present invention relates to a tracheostomy valve allowing a patient to speak which is intended to be attached to the end of a tracheostomy tube in order to regulate air flow through said tube. The valve is in a closed state when the patient with the tracheostomy valve exhales (and possibly speaks) and opens only when the patient inhales, allowing air to flow through the tracheostomy valve and through the tracheotomy tube to the patients lungs. The tracheostomy valve is specifically designed to facilitate the speech of the patient.

The closest prior art is considered to be WO 2009/018384 disclosing a tracheostomy valve comprising a valve body having a first inner end, a second outer end, and a passageway extending between said ends through the valve body allowing air to flow from and trough said first end to said second end. Outer in this context means facing away from the patient (i.e. from the tracheostomy tube) and inner means facing the patient. The first inner end thus is adapted to be attachable to the tracheostomy tube. A transverse flexible membrane is located within the passageway at a distance from said first end.

A rib is located in the passageway transversely to the direction of the air flow through said passageway in the vicinity of the outer end. A post is located at the center of the rib on the outer side of the rib. This post is intended to cooperate with a central hole in the membrane to hold the membrane in place. The rib has a slightly raised portion adjoining the post. A cap can be secured to the outer end of the valve having an opening with a diameter largely corresponding to the inner diameter of the passageway, said opening defining a rim. The cap is provided with a hub, radial spokes extending from said hub to said rim. The hub is provided with a hole designed to mate with said post. The rim is provided with a seating ring on the inner side (i.e. the side facing the patient when the cap is mounted) for supporting the periphery of the membrane. When the cap is attached, the central part of the membrane will be clamped between said hub and said raised portion on the rib. The seating ring is offset inwards relative to the inner surface of the hub.

When the cap is attached, the membrane thus is deformed so as to be preloaded into engagement with the seating ring intended to result in an uninterrupted positive seal between membrane and seating ring.

The general principle of preloading a membrane in a tracheotomy valve in this way is also disclosed in US-A-4259356.

A further document describing the general state of the art of the above kind of tracheostomy valves is EP 1377334 B1.

### Object of the invention.

The object of the present invention is to obtain a tracheotomy valve of the general kind described above which opens immediately upon inhalation and which closes immediately and positively at the end of inhalation. The air flow through valve also should be optimized. The valve should remain closed and sealed during exhalation and should not open until the next inhalation. One reason that this is important is to allow the patient to speak clearly. It is also desirable that the opening and the closing of the valve is fast and substantially noiseless. The valve as such also should minimize noise.

This object is achieved with a tracheostomy valve as set forth in the appended claims.

### Brief description of the drawings.

Fig 1 is a three-dimensional longitudinal section of an embodiment of the tracheotomy valve according to the invention seen from the outer side,
Fig 2 is a three-dimensional longitudinal section of an embodiment of the tracheotomy valve according to the invention seen from the inner side, the sectional surface being perpendicular to the sectional surface in Fig 1,
Fig 3 is a planar section A - A of the section of the valve in Fig 1 taken perpendicularly to the section in Fig 1,
Fig 4 is a section B - B of the valve in Fig 1, parallel to the section A - A, but slightly offset,

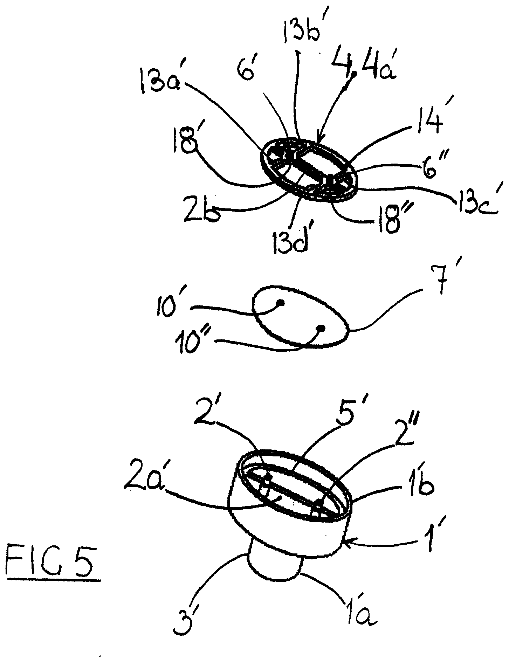

Fig 5 is an exploded three-dimensional view of another embodiment of the invention in which the membrane has an oval shape,

### Preferred embodiments of the invention

In the context of the present description and claims the expressions "inner " or "inwardly" indicate the part(s) or feature(s) being closest to, oriented towards or facing the patient. The expressions "outer" or "outwardly" indicate the part(s) being farthest away from, oriented away from or facing away from the patient when the valve is attached to a tracheotomy tube.

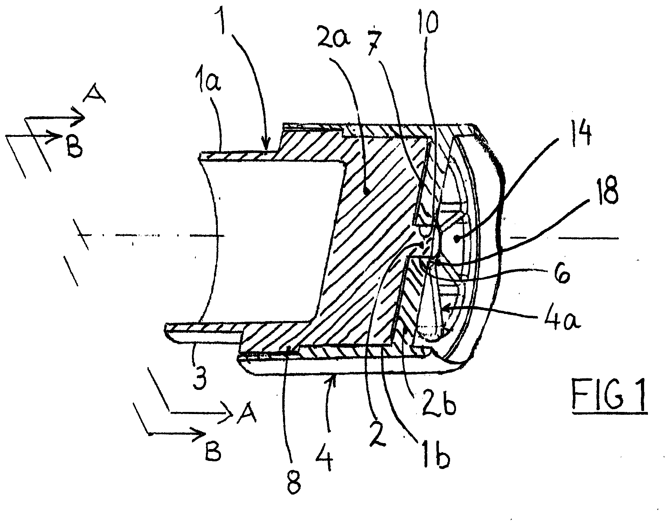

A first preferred embodiment of the invention is shown in Figs 1 - 4. This embodiment comprises a tracheostomy valve with a valve body (1) having two ends, a first inner end (1a), a second outer end (1b), and a passageway extending between said ends (1a, 1b) through the valve body allowing air to flow from and through said first end (1a) to and through said second end (1b). The passageway is circular in cross-section. The outside of the inner end (1a) is provided with a conical shape (3) to mate with a standard tracheotomy tube. A beam (2a) is arranged diametrically in the outer end (1b) of the valve body (1). A post (2) with a circular cross-section is arranged centrally and directed perpendicularly outwardly on the outer side of the beam (2a). The valve also comprises a circular membrane (7) placed on the outer side of the beam (2a). The membrane (7) has a circular hole (10) at the center having a diameter corresponding to the diameter of the post (2). The post (2) extends through the hole (10) in the membrane (7). The outer surface of the first beam (2a) is straight and planar.

The valve also comprises a cap (4) which is secured to the outer end (1b) of the valve body (1). The cap has a circular opening with a diameter largely corresponding to the inner diameter of the passageway. This opening has a circular rim (14). The cap is provided with a second beam (2b). The inner surface of said second beam (2b) is also straight and planar. The distance between the two beams (2a, 2b) corresponds to the thickness of the membrane (7). The membrane (7) thus is held between the first beam (2a) and the second beam (2b) (See Fig 1). The second beam (2b) is provided with a central hole (6) corresponding in size and shape to the post (2). The post (2) is engaged in this hole (6), thus further securing the membrane to the beams (2a, 2b). The rim is provided with a seating surface (5) along the inner periphery of the rim (14). The seating surface (5) faces inwardly towards the membrane for supporting the periphery of the membrane (7). The second beam (2b) is provided with a central hub (18) in which the hole (6) is formed. Ribs (spokes) (13 a - f) extend radially from the hub (18) to the rim (14). The second beam (2b), the ribs (13 a- f) and the rim (14) thus form a grid (4a).

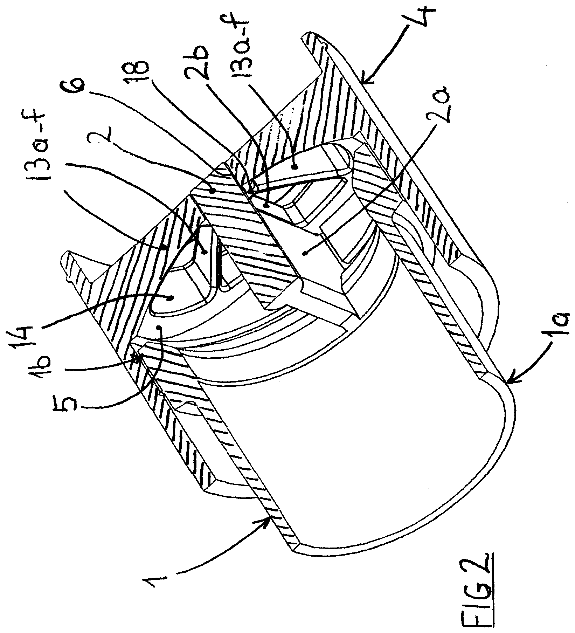

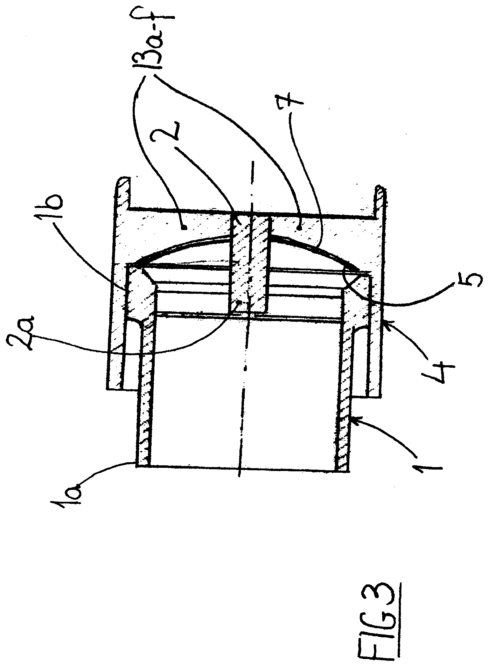

The seating surface (5) is arranged to coincide with the inner surface of the second beam (1b) at each end of the beam (2b), as for instance can be seen in Figs 2 and 3. The seating surface (5) on each side of said second beam (2b) is progressively and increasingly offset inwardly to reach a maximum distance from a transverse plane through the inner surface of the second beam (2b) after a quarter of the circumference of the circular rim (14). The seating surface (5) is then progressively and decreasingly offset outwardly to the second end of the beam (2b). If the longest distance between the second beam (2b) and the seating surface (5) is R, the maximal offset may for instance be 0.2R.

The inner surfaces (i. e. of the grid 4a) of the second beam (2b) and the ribs (13 a - f) are curved and the seating surface (5) merges smoothly and continuously with the inner surfaces of said ribs (13 a - f) and the inner surface of the second beam (2b). The inner surfaces of the radial ribs (13 a - f), the seating surface (5) and the inner surface of the second beam (2b) (i.e. the inner surface of the grid) can be said to generally follow the surface a cylindrical envelope that is oriented inwardly and has a generatrix located along the inner side of the second beam (2b).

Another way of visualizing the surface defined by the inner sides the grid would be the curved shape or periphery of a completely flexible circular membrane placed on top of the outer cylindrical surface of a cylinder. The apex or highest point would be located on and along the inner surface of the second beam (2b).

The inner shape of the grid (4a) need not necessarily be cylindrical. A parabolic or hyperbolic shape with an apical generatrix similarly located along the second beam (2b) could also be used as well as any other similar curve.

Each one of the beam (2b), the ribs (13 a - f) and the seating surface (5) thus will support the membrane (7) along its entire length. It should be noted that membrane (7) has been left out in Fig 2 in order to better illustrate the curved shapes of the inner side of the second beam (2b), of the inner surface of the ribs (13 a - f) and of the seating surface (5).

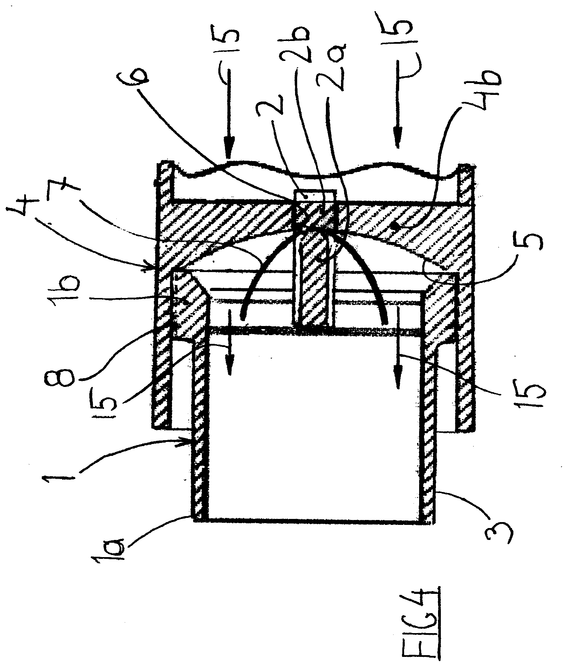

Figs 3 and 4 illustrate the function of the valve.

In Fig 3 the valve is shown in a closed state. The section is taken through the post (2). The membrane (7) located on the planar outer surface of the first beam is given a vaulted shape defined the grid as described above. Since the membrane (7) normally is planar, the membrane when mounted will be preloaded against the curved inside of the grid (4a) and consequently into a positively closed and sealing position against the seating ring (5) at all times except when the patient inhales. The general curvature of the inner side of the grid also will shape and hold the membrane in such a way that the risk for any folds or wrinkles at the edge of the membrane is minimized, the membrane being flexed in only one direction (the direction of the generatrix) over its entire width.

In Fig 4, the valve is shown in the open state, i. e. when the patient inhales. The direction of the air flow is indicated by the arrows (15). This view is parallel to, but slightly offset from the section in Fig 3 and consequently the first beam (2a) can be seen in cross-section. As illustrated, the membrane (7) bends away from the seating surface (5) in two segments along the two sides of the outer surface of the first beam (2a). Since the distance between the two beams (2a, 2b) is equal to the thickness of the membrane (7), the entire diameter of the membrane will be held in contact with the outer surface of the first beam (2a) along the entire length of said beam. The edges of the first beam (2a) thus will positively guide the flexing movement of the respective membrane segment. The membrane (7) also is held without constraints in the space between the first and second beam (2a, 2b). The first feature will minimize the influence of any folds, wrinkles or other irregularities in the membrane (7) and the second will minimize the risk for such folds, wrinkles or irregularities caused by clamping or otherwise constraining the membrane (7). This consequently means that the membrane easily will open to the maximum extent possible. As indicated in Fig 4, the edges of the outer surface of the first beam (2a) preferably are rounded, which further will facilitate the flexing of the membrane (7) as well as improve the aerodynamic properties of the passageway. When the membrane flexes towards its closed position the membrane might progressively follow the curved grid from the central part to the seating ring, thus improving the noise characteristics.

Since the above embodiment is circular in cross-section, the valve body and the cap may be provided with orienting means ensuring that the beams (2a, 2b) are located along each other when the valve is assembled. In this particular embodiment the orienting means comprise splines (8) located in one or several locations along the periphery.

In an alternative embodiment of the invention, the passageway and the membrane (7') are generally elliptical in cross-section and the valve body (1') is adapted to conform to this shape. The first beam (2'a) is then arranged along the major axis of the ellipse. In this case it is important to prevent the membrane (7') from rotating and consequently the outer side of the first beam is provided with at least two posts (2', 2") cooperating with corresponding holes (10', 10") in the membrane (7'). The posts (2', 2") could for instance be located so as to divide the length of the first beam (2a') into three equal parts or alternatively be located in the foci of the ellipse.

The opening in the cap (4') also is elliptical with the second beam (2b') located at the major axis of the ellipse. The second beam (2b') is provided with holes (6', 6") corresponding to the posts (2', 2"). The ribs (13'a - d) forming the grid can be arranged in different ways relative to the beam (2b'). Thus, as illustrated, all ribs (13'a - d) could be arranged perpendicularly to the beam (2b') or the ribs on the side of the posts facing the respective small ends of the ellipse could be arranged more or less radially centered on the respective post and the ribs located in the space between the posts could be arranged perpendicularly to the beam.

In this embodiment, cap 4' does not include any other features than the grid 4a'except that the outside is adapted to fit into the passageway.

The inside of the grid (4a') is shaped cylindrically, parabolically, hyperbolically or otherwise in same way as in the above first embodiment.

One advantage with an elliptical cross-section is that the height of the valve is minimized if the major axis of the valve is oriented transversely to the patient's throat, the valve thus being more comfortable to wear.

Within the scope of the invention the membrane also could have a superelliptic shape. The superelliptic shape could even verge towards a rectangular shape.

In all other respects this embodiment corresponds to the first embodiment although being adapted to the elliptical/superelliptical shape where necessary.

Several variations of the invention are possible within the scope of the appended claims. For instance in the above embodiments, the posts are located on the first beam and the holes in the second beam. It is however quite possible to locate the posts on the second beam and the holes in the first beam.

It should be noted that the valve in common with tracheostomy valves in general also may be provided with connections for a ventilating machine and/or an oxygen supply and/or with filters and/or with a shower/heat shield. Furthermore, the ribs may be provided with a cross-section shaped as a narrow, symmetrical airfoil in order to improve the aerodynamics of the valve.

## Claims

1. Tracheostomy valve comprising a valve body (1, 1') having a first inner end (1a, 1a'), a second outer end (1b, 1b'), and a passageway extending between said ends (1a, 1a'; 1b, 1b') through the valve body allowing air to flow from and through said first end (1a, 1a') to and through said second end (1b, 1b',), a first beam (2a, 2a') located in the passageway transversely to the direction of the air flow through said passageway at the outer end, a transverse flexible membrane (7, 7') located within the passageway on the outer surface of said beam (2a, 2a'), said valve further comprising a cap (4, 4') securable to the outer end (1b, 1b') of the valve having an opening with a size largely corresponding to the inner size of the passageway, said opening being defined by a circumferential rim (14, 14'), said cap being provided with a second beam (2b, 2b) located in parallel along said first beam (2a', 2a), said membrane (7, 7') being held between said first beam (2a, 2a') and said second beam (2b, 2b'), said first beam (2a, 2a') and said second beam (2b, 2b') being provided with securing means (2,2'; 6,6') for additionally securing said membrane (7, 7') between said first beam (2a, 2a') and said second beam (2b, 2b'), said rim being provided with a seating surface (5, 5') facing inwardly for supporting the periphery of said membrane (7, 7'), said second beam being provided with ribs (13 a - f, 13'a-d) extending from said second beam (2b, 2b') to said rim (14, 14'), said second beam (2b, 2b') together with said ribs (13 a - f, 13'a-d) and said rim (14, 14') forming a grid (4a, 4a'), said membrane (7, 7') being deformed and preloaded into a closed position towards said seating surface (5), **characterized in that** the outer surface of said first beam (2a, 2a') is straight and planar.

2. Tracheostomy valve according to claim 1, **characterized in that** the inner surface of said second beam (2b, 2b') is straight and planar and **in that** the space between said first (2a, 2a') and said second beam (2b, 2b') corresponds to the thickness of said membrane (7).

3. Tracheostomy valve according to claim 1 or 2, **characterized in that** seating surface (5, 5') on each side of said second beam (2b, 2b') is progressively and increasingly offset inwardly from a plane along the inner side of said second beam from zero distance at a first end of said beam(2b) to reach a maximum distance, said seating surface (5) being progressively and decreasingly offset outwardly to zero distance at said plane at the second end of said beam (2b, 2b'), said seating surface further merging smoothly and continuously with the inner surfaces of said ribs (13a-f, 13'a-d).

4. Tracheostomy valve according to claim 3, **characterized in that** the inside of said grid (4a, 4a') is curved and that this curvature is defined and generated by a straight generatrix which in one position coincides longitudinally with the inner surface of the second beam (2b, 2b').

5. Tracheostomy valve according to claim 4, **characterized in that** said curvature is cylindrical.

6. Tracheostomy valve according to claim 4, **characterized in that** the curvature is hyperbolic, the apex of the hyperbel in question coinciding with the inner surface of said second beam.

7. Tracheostomy valve according to claim 4, **characterized in that** the curvature is parabolic, the apex of the parable in question coinciding with the inner surface of said second beam.

8. Tracheostomy valve according to any of the preceding claims **characterized in that** the edges of the outer surface of the first beam (2a,2a') are rounded.

9. Tracheostomy valve according to any of the preceding claims **characterized in that** said ribs (13a-f; 13'a-d) are provided with a cross-section shaped as a narrow, symmetrical airfoil.

10. Tracheostomy valve according to any one of the preceding claims, **characterized in that** said first beam (2a, 2a') is provided with at least one post (2, 2', 2") directed perpendicularly and outwardly on the outer side of the beam (2a, 2a') and cooperating with at least one hole (6, 6',6") in the membrane (7, 7') having a diameter corresponding to the diameter of the posts (2, 2',2 ") in order to further secure the membrane in the valve.

## Patentansprüche

1. Tracheostomieventil umfassend einen Ventilkörper (1,1') mit einem ersten inneren Ende (1a, 1a'), einem zweiten äußeren Ende (1b, 1b') und einem Durchgang, der sich zwischen den Enden (1a, 1a'; 1b, 1b') durch den Ventilkörper hindurch erstreckt, um zu ermöglichen, dass Luft von dem ersten Ende (1a, 1a') und durch dieses hindurch bis zu dem zweiten Ende (1b, 1b',) und durch dieses hindurch strömen kann, einen ersten Träger (2a, 2a'), der in dem Durchgang quer zur Richtung der durch den Durchgang an dem äu-ßeren Ende hindurch strömenden Luft angeordnet ist, eine quergehende flexible Membran (7, 7'), die in dem Durchgang an der Außenfläche des Trägers (2a, 2a') angeordnet ist, wobei das Ventil ferner eine an dem äußeren Ende (1b, 1b') des Ventils befestigbare Kappe (4, 4') umfasst, die eine Öffnung mit einer Größe, die weitgehend der inneren Größe des Durchgangs entspricht, aufweist, wobei die Öffnung durch einen umfänglichen Rand (14, 14') definiert ist, wobei die Kappe mit einem zweiten Träger (2b, 2b) versehen ist, der parallel zu dem ersten Träger (2a', 2a) angeordnet ist, wobei die Membran (7, 7') zwischen dem ersten Träger (2a, 2a') und dem zweiten Träger (2b, 2b') gehalten ist, wobei der erste Träger (2a, 2a') und der zweite Träger (2b, 2b') mit Befestigungsmitteln (2,2'; 6,6') versehen sind, um die Membran (7,7') zwischen dem ersten Träger (2a, 2a') und dem zweiten Träger (2b, 2b') weiter zu befestigen, wobei der Rand mit einer Sitzfläche (5, 5') versehen ist, die nach innen gewandt ist, um den Umfang der Membran (7, 7') zu unterstützen, wobei der zweite Träger mit Stegen (13 a - f, 13'a-d) versehen ist, die sich von dem zweiten Träger (2b, 2b') bis zum Rand (14, 14') erstrecken, wobei der zweite Träger (2b, 2b') zusammen mit den Stegen (13 a - f, 13'a-d) und dem Rand (14, 14') ein Gitter (4a, 4a') bildet, wobei die Membran (7, 7') verformt ist und in einer geschlossenen Position gegen die Sitzfläche (5) vorgespannt ist, **dadurch gekennzeichnet, dass** die Außenfläche des ersten Trägers (2a, 2a') gerade und eben ist.

2. Tracheostomieventil nach Anspruch 1, **dadurch gekennzeichnet, dass** die Innenfläche des zweiten Trägers (2b, 2b') gerade und eben ist, und dass der Zwischenraum zwischen dem ersten (2a, 2a') und dem zweiten Träger (2b, 2b') der Dicke der Membran (7) entspricht.

3. Tracheostomieventil nach Anspruch 1 oder 2, **dadurch gekennzeichnet, dass** die Sitzfläche (5, 5') an jeder Seite des zweiten Trägers (2b, 2b') schrittweise und zunehmend von einer Ebene entlang der Innenseite des zweiten Trägers von einem Abstand Null an einem ersten Ende des Trägers (2b) nach innen versetzt ist, um einen Höchstabstand zu erreichen, wobei die Sitzfläche (5) schrittweise und zunehmend bis zum Abstand Null bei der Ebene am zweiten Ende des Trägers (2b, 2b') nach außen versetzt ist, wobei die Sitzfläche ferner gleichmäßig und kontinuierlich mit den Innenflächen der Ränder (13a - f, 13'a-d) zusammenläuft.

4. Tracheostomieventil nach Anspruch 3, **dadurch gekennzeichnet, dass** die Innenseite des Gitters (4a, 4a') gekrümmt ist, und dass diese Krümmung durch eine gerade Mantellinie definiert und erzeugt ist, welche in einer Position mit der Innenfläche des zweiten Trägers (2b, 2b') in Längsrichtung zusammenfällt.

5. Tracheostomieventil nach Anspruch 4, **dadurch gekennzeichnet, dass** die Krümmung zylindrisch ist.

6. Tracheostomieventil nach Anspruch 4, **dadurch gekennzeichnet, dass** die Krümmung hyperbolisch ist, wobei das Apex der betreffenden Hyperbel mit der Innenfläche des zweiten Trägers zusammenfällt.

7. Tracheostomieventil nach Anspruch 4, **dadurch gekennzeichnet, dass** die Krümmung parabolisch ist, wobei das Apex der betreffenden Parabel mit der Innenfläche des zweiten Trägers zusammenfällt.

8. Tracheostomieventil nach einem der vorgehenden Ansprüche, **dadurch gekennzeichnet, dass** die Kanten der Außenfläche des ersten Trägers (2a, 2a') gerundet sind.

9. Tracheostomieventil nach einem der vorgehenden Ansprüche, **dadurch gekennzeichnet, dass** die Stege (13a-f, 13'a-d) mit einem Querschnitt versehen sind, der als eine schmale, symmetrische Tragfläche gebildet ist.

10. Tracheostomieventil nach einem der vorgehenden Ansprüche, **dadurch gekennzeichnet, dass** der erste Träger (2a, 2a') mit mindestens einem Pfosten (2, 2', 2") versehen ist, der an der anderen Seite des Trägers (2a, 2a') senkrecht und nach außen gerichtet ist und mit mindestens einem Loch (6, 6', 6") in der Membran (7, 7') zusammenwirkt, das einen Durchmesser aufweist, der dem Durchmesser der Pfosten (2, 2', 2") entspricht, um die Membran in dem Ventil weiter zu befestigen.

## Revendications

1. Soupape de trachéotomie comprenant un corps de soupape (1,1') ayant une première extrémité intérieure (1a, 1a'), et une deuxième extrémité extérieure (1b, 1b'), et un passage s'étendant entre lesdites extrémités (1a,1a'; 1b, 1b') à travers le corps de soupape permettant à l'air de s'écouler à partir de ladite première extrémité (1a, 1a') et à travers celle-ci et vers et à travers ladite deuxième extrémité (1b, 1b',), une première poutre (2a, 2a') située dans le passage transversalement à la direction de l'écoulement d'air à travers ledit passage à l'extrémité extérieure, une membrane flexible transversale (7, 7') située dans le passage sur la surface extérieure de ladite poutre (2a, 2a'), ladite soupape comprenant en outre un capuchon (4, 4') pouvant être fixé à l'extrémité extérieure (1b, 1b') de la soupape ayant une ouverture avec une taille correspondant en grande partie à la taille intérieure du passage, ladite ouverture étant définie par un rebord circonférentiel (14, 14'), ledit capuchon étant muni d'une deuxième poutre (2b, 2b) située en parallèle le long de ladite première poutre (2a', 2a), ladite membrane (7, 7 ') étant maintenue entre ladite première poutre (2a, 2a') et ladite deuxième poutre (2b, 2b'), ladite première poutre (2a, 2a') et ladite deuxième poutre (2b, 2b') étant munies de moyens de fixation (2,2'; 6,6') pour fixer en outre ladite membrane (7, 7') entre ladite première poutre (2a, 2a') et ladite deuxième poutre (2b, 2b'), ledit rebord étant pourvu d'une surface d'appui (5, 5') orientée vers l'intérieur pour supporter la périphérie de ladite membrane (7, 7'), ladite deuxième poutre étant pourvue de nervures (13 a - f, 13'a-d) s'étendant depuis ladite deuxième poutre (2b, 2b') audit rebord (14, 14'), ladite deuxième poutre (2b, 2b') avec lesdites nervures (13 a - f, 13'a-d) et ledit rebord (14, 14') formant une grille (4a, 4a'), ladite membrane (7, 7') étant déformée et précontrainte dans une position fermée vers ladite surface d'appui (5), **caractérisée en ce que** la surface extérieure de ladite première poutre (2a, 2a') est droite et plane.

2. Soupape de trachéotomie selon la revendication 1, **caractérisée en ce que** la surface intérieure de ladite deuxième poutre (2b, 2b') est droite et plane, et **en ce que** l'espace entre lesdites première (2a, 2a') et deuxième poutre (2b, 2b') correspond à l'épaisseur de ladite membrane (7).

3. Soupape de trachéotomie selon la revendication 1 ou 2, **caractérisée en ce que** la surface d'appui (5, 5') de chaque côté de ladite deuxième poutre (2b, 2b') est progressivement et de plus en plus décalée vers l'intérieur à partir d'un plan le long du côté intérieur de ladite deuxième poutre à partir d'une distance nulle à une première extrémité de ladite poutre (2b) pour atteindre une distance maximale, ladite surface d'appui (5) étant progressivement et de plus en plus décalée vers l'extérieur d'une distance nulle audit plan à la deuxième extrémité de ladite poutre (2b, 2b'), ladite surface d'appui fusionnant de façon lisse et continue avec les surfaces intérieures desdites nervures (13a - f, 13'a-d).

4. Soupape de trachéotomie selon la revendication 3, **caractérisée en ce que** l'intérieur de ladite grille (4a, 4a') est courbé, et **en ce que** cette courbure est définie et générée par une génératrice droite qui, dans une position, coïncide longitudinalement avec la surface intérieure de la deuxième poutre (2b, 2b').

5. Soupape de trachéotomie selon la revendication 4, **caractérisée en ce que** ladite courbure est cylindrique.

6. Soupape de trachéotomie selon la revendication 4, **caractérisée en ce que** la courbure est hyperbolique, le sommet de l'hyperbole en question coïncidant avec la surface intérieure de ladite deuxième poutre.

7. Soupape de trachéotomie selon la revendication 4, **caractérisée en ce que** la courbure est parabolique, le sommet de la parabole en question coïncidant avec la surface intérieure de ladite deuxième poutre.

8. Soupape de trachéotomie selon l'une quelconque des revendications précédentes, **caractérisée en ce que** les bords de la surface extérieure de la première poutre (2a, 2a ') sont arrondis.

9. Soupape de trachéotomie selon l'une quelconque des revendications précédentes, **caractérisée en ce que** lesdites nervures (13a-f, 13'a-d) sont munies d'une section en forme de profil aérodynamique étroit et symétrique.

10. Soupape de trachéotomie selon l'une quelconque des revendications précédentes, **caractérisée en ce que** ladite première poutre (2a, 2a') est pourvue d'au moins un poteau (2, 2', 2") dirigé perpendiculairement et vers l'extérieur du côté extérieur de la poutre (2a, 2a') et coopérant avec au moins un trou (6, 6', 6") dans la membrane (7, 7') ayant un diamètre correspondant au diamètre des poteaux (2, 2', 2") afin de sécuriser davantage la membrane dans la soupape.
